# EUROPÄISCHE PATENTSCHRIFT

(11) **EP 0 586 795 B1**
(45) Veröffentlichungstag und Bekanntmachung des Hinweises auf die Patenterteilung: **14.05.1997**
(21) Anmeldenummer: 93109096.3
(22) Anmeldetag: 07.06.1993
(51) Int. Cl.: G01B 11/30

(54) **Verfahren und Vorrichtung zur berührungslosen Überprüfung der Oberflächenrauhigkeit von Materialien**
Procedure and device for the contactless determination of the roughness of surface of objects
Dispositif et procédé pour la détermination sans contact de la rugosité de surface de matériaux

(30) Priorität: 09.09.1992 DE 4230068
(43) Veröffentlichungstag der Anmeldung: 16.03.1994
(73) Patentinhaber: TZN Forschungs- und Entwicklungszentrum Unterlüss GmbH, D-29345 Unterlüss (DE)
(72) Erfinder: Giet, Johannes, Dr., W-3104 Unterlüss (DE); Meyer, Walter, W-3104 Unterlüss (DE)
(74) Vertreter: Behrend, Rainer

(56) Entgegenhaltungen:
- EP-A- 0 486 219
- WO-A-89/07235
- PATENT ABSTRACTS OF JAPAN vol. 10, no. 250 (P-491)(2306) 28. August 1986 & JP-A-61 077 709 (OMRON TATEISI ELECTRONICS CO) 21. April 1986
- PATENT ABSTRACTS OF JAPAN vol. 9, no. 299 (P-408)(2022) 27. November 1985 & JP-A-60 135 707 (MATSUSHITA DENKO K.K.) 19. Juli 1985
- PATENT ABSTRACTS OF JAPAN vol. 11, no. 49 (P-547)(2496) 14. Februar 1987 & JP-A-61 217 708 (NIPPON PAINT CO LTD) 27. September 1986
- PATENT ABSTRACTS OF JAPAN vol. 6, no. 121 (P-126)(999) 6. Juli 1982 & JP-A-57 049 805 (MATSUSHITA DENKO K.K.) 24. März 1982

## Beschreibung

Die Erfindung bezieht sich auf ein Verfahren zur berührungslosen Überprüfung der Oberflächenrauhigkeit von Materialien, wie es durch die Merkmale des Oberbegriffs des Anspruchs 1 näher charakterisiert ist. Die Erfindung bezieht sich ferner auf eine optische Reflektionsmeßvorrichtung zur Durchführung dieses Verfahrens (Anspruch 3).

Optische Verfahren zur Messung und Überprüfung der Oberflächenrauhigkeit sind seit langem bekannt. Insbesondere ist es bekannt, zur Rauhigkeitsmessung von Papieroberflächen beim jeweiligen Glanzwinkel die Intensität des reflektierten und gestreuten Lichtes zu messen und diese als Maß für die Oberflächenrauhigkeit heranzuziehen. Bei einem anderen Verfahren wird als charakterisisticher Wert für die Oberflächenrauhigkeit die Halbwertsbreite des gestreuten Lichtes herangezogen.

Bekannt ist aus EP 0 486 219 ferner, mit Hilfe von Test-Hilfsmustern aus den resultierenden reflektierten Lichtsignalen Aussagen über die Oberflächenrauhigkeit zu treffen. Dabei wird die zu bearbeitende Fläche zeilenartig abgetastet. Auftretende Signal-Verzerrungen zwischen Testsignal und reflektiertem Signal tragen die Information der Oberflächenrauhigkeit .

Bei der JP-A-57049805 (Ab) wird die Fläche mit Hilfe einer einzelnen Linie, die auf das Material projiziert wird, abgetastet. Auch hier erfolgt die Analysierung der Rauhigkeit über eine Fourieranalyse, nachdem eine Veränderung zwischen Test- und reflektiertem Signal ermittelt wurde.

Alle diese Verfahren besitzen den Nachteil, daß sie relativ ungenau arbeiten und nicht ohne weiteres zur Messung anderer Materialien (Leder, Schaumstoff, Schmirgelpapier etc.) herangezogen werden können.

Bekanntgeworden sind ferner Verfahren zur Oberflächenrauhigkeitsprüfung, die mit berührenden Prüfgeräten arbeiten. Derartige Verfahren weisen den Nachteil auf, daß sie sehr zeitintensiv arbeiten und nur zur Stichprobenprüfung, nicht aber zu einer kontinuierlichen Prüfung der Oberflächenrauhigkeit geeignet sind.

Der Erfindung liegt daher die Aufgabe zugrunde, ein berührungslos arbeitendes optisches Verfahren zur Bestimmung der Oberflächenrauhigkeit anzugeben, welches reproduzierbare Meßwerte ergibt und weitgehend universell zur Prüfung der Oberflächenrauhigkeit unterschiedlicher Materialien benutzt werden kann. Ferner soll eine Vorrichtung zur Durchführung dieses Verfahrens offenbart werden.

Erfindungsgemäß wird die vorstehend erwähnte Aufgabe durch die Merkmale des kennzeichnenden Teiles des Anspruchs 1 gelöst. Eine erfindungsgemäße Vorrichtung offenbart Anspruch 3.

Im wesentlichen liegt der Erfindung also der Gedanke zugrunde, einen frequenzselektiven Bereich der Bildenergie als Maß für die Oberflächenrauhigkeit zu verwenden. Dabei werden zur Rauhigkeitsbestimmung nicht die gemessenen Intensitätswerte direkt herangezogen, sondern das mit Hilfe einer Fouriertransformation aus diesen Werten ermittelte Ortsfrequenzspektrum bzw. bestimmte aussagekräftige Bereiche des Ortsfrequenzspektrums. Es hat sich nämlich gezeigt, daß sich für vorgegebene Referenzmuster charakteristische Ortsfrequenzspektren ergeben, so daß Abweichungen von diesen (Soll-) Spektren sehr genau gemessen werden können. Vor allem, wenn die Oberfläche des Materials ausgeprägte Kanten aufweist, erhält man charakteristische Spektrallinien im höheren Frequenzbereich.

Weitere Einzelheiten und Vorteile der Erfindung werden im folgenden anhand von in Figuren dargestellten Ausführungsbeispielen beschrieben. Es zeigen:
- Fig.1: eine Reflektionsmeßvorrichtung mit Seitenlichtbeleuchtung; und
- Fig.2: eine entsprechende Meßvorrichtung mit Ringlichtbeleuchtung.

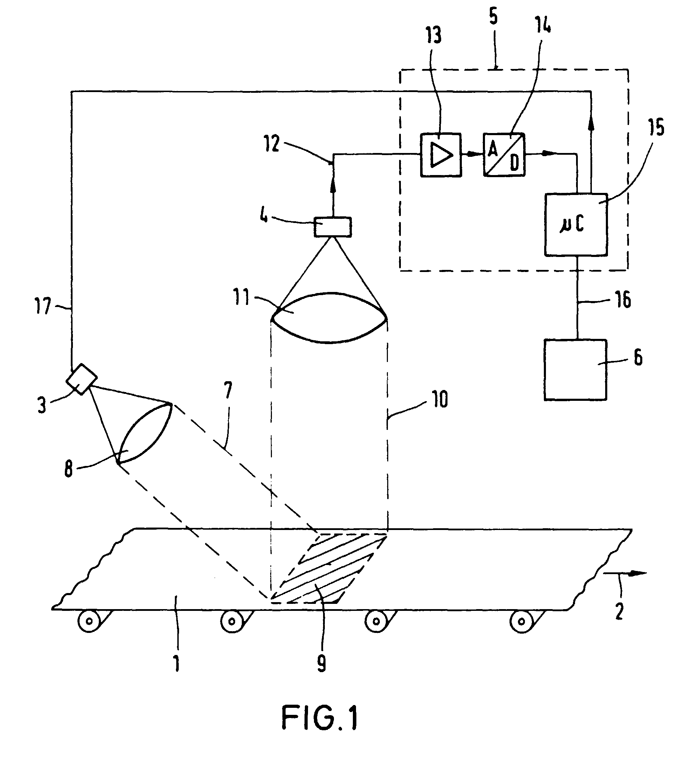

In Fig.1 wird mit 1 eine auf ihre Oberflächenrauhigkeit zu überprüfende Materialbahn, z. B. aus Kunstleder, bezeichnet, die sich in die mit dem Bezugszeichen 2 angedeutete Richtung bewegen möge. Über der zu überprüfenden Materialbahn 1 befindet sich eine optische Reflektionsvorrichtung, welche im wesentlichen aus einer Lichtquelle 3, einer Sensoreinheit 4, einer Auswerteeinheit 5 und einer Ein-/Ausgabevorrichtung 6 besteht.

Das von der Lichtquelle (Seitenlichtquelle) 3 erzeugte Lichtbündel 7 gelangt über eine Optik 8 auf einen vorgegebenen Bereich 9 der zu überprüfenden Materialbahn 1, wird dort reflektiert und gestreut, und das entsprechende reflektierte und gestreute Lichtbündel 10 gelangt über eine weitere Optik 11 in die Sensoreinheit 4. Dabei wird als Sensoreinheit 4 ein CCD-Zeilen- oder CCD-Matrixsensor verwendet.

Die Sensorsignale werden anschließend über eine elektrische Leitung 12 der Auswerteeinheit 5 zugeführt. In dieser werden die Signale zunächst in einem Verstärker 13 verstärkt, dann in einem Analog-/Digitalwandler 14 in entsprechende digitale Werte transformiert, schließlich in einem Mikrorechner 15 weiterverarbeitet und das Ergebnis von der Ein-/Ausgabevorrichtung angezeigt.

Zur Verarbeitung der digitalisierten Meßwerte werden die ortsabhängigen Intensitätswerte f(x,y) (wobei x und y die Ortskoordinaten und f(x,y) die entsprechende von diesen Koordinaten abhängige Intensitätsfunktion bedeuten) mit Hilfe des Rechners 15 über eine 2-dimensionale Fouriertransformation in entsprechende Werte des Ortsfrequenzspektrums |U(fx,fy)|² (wobei mit fₓ die Ortsfrequenz in x-Richtung und mit f_{y} die Ortsfrequenz in y-Richtung bezeichnet ist) transformiert und mit den entsprechenden Werten einer Oberfläche vorgegebener Rauhigkeit verglichen. Da bei einer idealen Oberflächenstruktur ein für die Rauhigkeit charakteristisches Ortsfrequenzspektrum vorliegt, lassen sich Abweichungen der Rauhigkeit von der Sollwertoberfläche sehr genau ermitteln.

Um die Lichtquelle 3 und die Sensoreinheit 4, je nach Aufgabe, auch spektral selektiv der zu überprüfenden Materialbahn anpassen zu können, ist zwischen Auswerteeinheit 5 und Lichtquelle eine entsprechende elektrische Steuerleitung 17 vorgesehen.

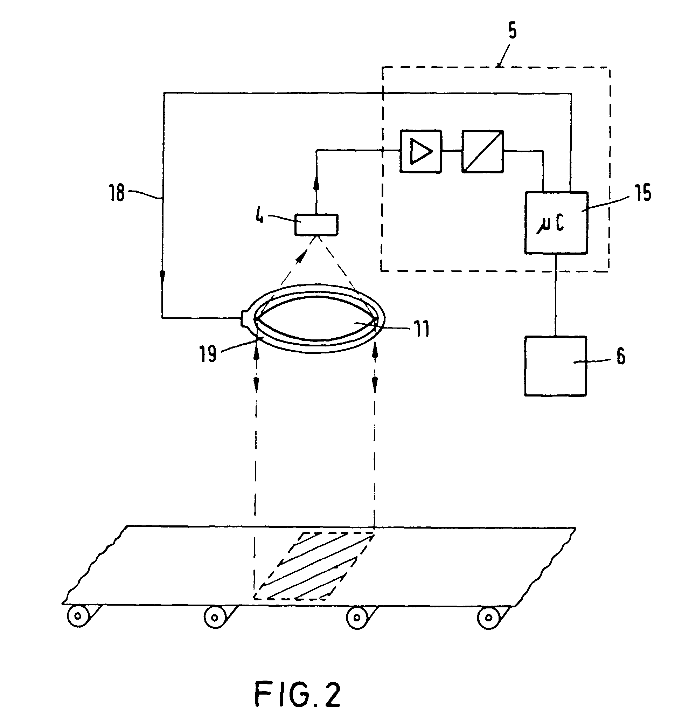

Die in Fig.2 dargestellte Reflektionsmeßvorrichtung unterscheidet sich von der in Fig.1 dargestellten Vorrichtung lediglich dadurch, daß als Lichtquelle eine Ringlichtquelle (mit nicht dargestellter Optik) benutzt wird.

Das erfindungsgemäße Verfahren hat sich besonders zur Beurteilung der Narbtiefe von Echt- oder Kunstleder sowie zur Kontrolle von Schmirgelpapier in der Fließfertigung, aber z.B. auch bei der Kontrolle der Porengröße von Schaumstoffen, bewährt.

### Bezugsliste

- 1: zu untersuchende Materialbahn
- 2: Richtungspfeil
- 3: Lichtquelle, Seitenlichtquelle
- 4: Sensoreinheit
- 5: Auswerteeinheit
- 6: Ein-/Ausgabevorrichtung
- 7: Lichtbündel
- 8: Optik
- 9: bestrahlter Bereich
- 10: gestreutes Lichtbündel
- 11: Optik
- 12: elektr. Leitung
- 13: Verstärker
- 14: Analog-/Digitalwandler
- 15: Mikrorechner ( µC)
- 16: elektr. Leitung
- 17: elektr. Leitung
- 18: elektr. Leitung
- 19: Ringlichtquelle

## Patentansprüche

1. Verfahren zur berührungslosen Überprüfung der Oberflächenrauhigkeit von Materialien (1) im Hinblick auf Abweichungen von einer Soll-Oberflächenrauhigkeit mit Hilfe einer optischen Reflektionsmeßvorrichtung und einer Fourier-Transformation, **dadurch gekennzeichnet**, daß
- in einem vorgegebenen Bereich (9) Intensitätswerte gemessen werden
- diese Intensitätswerte über eine 2 D-Fourier-Transformation in ein zugeordnetes Ortsfrequenzspektrum transformiert werden
- danach die durch die Rauhigkeit des Materials bedingten charakteristischen Spektralanteile im Ortsfrequenzspektrumanalysiert und
- mit der vorgegebenen, für die Soll-Oberflächenrauhigkeit charakteristischen Werten verglichen werden, wobei ein frequenzselektiver Bereich einer Bildenergie als Maß für die Oberflächenrauhigkeit genutzt wird.

2. Verfahren nach Anspruch 1, **dadurch gekennzeichnet**, daß zur Analyse charakteristische Spektrallinien im höheren Frequenzbereich herangezogen werden.

3. Optische Reflektionsmeßvorrichtung zur Durchführung des Verfahrens nach Anspruch 1, mit einer Lichtquelle (3, 19) zur Beleuchtung des zu untersuchenden Materials (1), einer Sensoreinheit (4) zum Empfang eines von dem Material (1) gestreuten Lichtbündels (10) und einer Auswerteeinheit
(5), die einen Rechner (15) zur Ermittlung der Rauhigkeit enthält, wobei der Rechner (5), die über einen CCD Matrix- oder Zeilensensor (4) empfangenen Intensitätswerte mittels 2-D-Fourier-Transformation in entsprechende Ortsfrequenzspektren transformiert, diese Werte mit den charakteristischen Ortsfrequenzspektren des vorbenen Referenzmusters vergleicht und die auf Grund dieses Vergleiches ermittelten Ergebnisse an eine Ein-/Ausgabevorrichtung (6) weiterleitet.

4. Vorrichtung nach Anspruch 3, **dadurch gekennzeichnet**, daß als Lichtquelle (3,19) eine Kaltlichtquelle verwendet wird.

5. Vorrichtung nach Anspruch 3 oder 4, **dadurch gekennzeichnet**, daß als Lichtquelle eine Ringlichtquelle (19) verwendet wird.

6. Vorrichtung nach Anspruch 3, **dadurch gekennzeichnet**, daß die Lichtquelle (3) und die Sensoreneinheit (4) spektral selektiv zu den überprüfenden Materialien (1) angeordnet sind.

## Claims

1. Method for contactless determination of the surface roughness of materials (1) in order to determine deviations from a required surface roughness using an optical reflection measuring device and a Fourier transformation, characterised by the fact that
-- intensity values are measured in a preselected zone (9),
-- these intensity values are transformed by means of a 2 D Fourier transformation into an associated spatial frequency spectrum,
-- the characteristic spectral parts produced by the roughness of the material are analysed over the spatial frequency spectrum and
-- are compared with the preselected value characteristic of the surface roughness, in which process a frequency-selective zone of the image energy is used as a measure of the surface roughness.

2. Method in accordance with Claim 1, characterised by the fact that for the analysis, use is made of characteristic spectral lines in the higher frequency range.

3. Optical reflection measuring device for carrying out the method described in Claim 1, using a light source (3,19) for illuminating the material (1) to be examined, a sensor unit (4) for receiving a beam (10) diffused from the material (1), and an evaluation unit (5) containing a computer (15) for determining the roughness, by which said computer the intensity values received by a CCD matrix or line sensor (4) are transformed by 2 D Fourier transformation into corresponding spatial frequency spectra, these values being compared with the characteristic spatial frequency spectra of a preselected reference pattern and the results obtained by said comparison being transmitted to an input/output device (6).

4. Device in accordance with Claim 3, characterised by the fact that the light source (3,19) used comprises a cold light source.

5. Device in accordance with Claim 3 or 4, characterised by the fact that the light source used consists of an annular light source (19).

6. Device in accordance with Claim 3, characterised by the fact that the light source (3) and the sensor unit (4) are arranged in a spectrally selective manner in relation to the materials (1) to be checked.

## Revendications

1. Procédé de vérification sans contact de la rugosité de surface de matériaux (1) en ce qui concerne les écarts par rapport à une rugosité de surface de consigne à l'aide d'un dispositif de mesure optique par réflexion et d'une transformation de Fourier, caractérisé en ce que
- dans une zone (9) donnée sont mesurées des valeurs d'intensité
- ces valeurs d'intensité sont transformées par une transtormation de Fourier 2 D dans un spectre de fréquences locales associé
- ensuite les fractions spectrales caractéristiques conditionnées par la rugosité du matériau, sont analysées dans le spectre de fréquences locales et
- sont comparées aux valeurs caractéristiques données pour la rugosité de surface de consigne, un domaine à sélection de fréquence d'une énergie d'image étant utilisé comme critère pour la rugosité de surface.

2. Procédé selon la revendication 1, caractérisé en ce qu'on utilise pour l'analyse des lignes spectrales caractéristiques dans la gamme de fréquences supérieure.

3. Dispositif de mesure optique de réflexion pour la mise en oeuvre du procédé selon la revendication 1 avec une source lumineuse (3, 19) pour éclairer le matériau (1) à analyser, une unité à capteur (4) pour la réception d'un faisceau lumineux (10) dispersé par le matériau (1) et une unité d'exploitation (5), qui contient un ordinateur (15) pour la détermination de la rugosité, l'ordinateur (15) transformant les valeurs d'intensité, reçues par une matrice CCD ou capteur de lignes (4), au moyen d'une transformation de Fourier 2 D, dans des spectres de fréquences locales correspondants, comparant ces valeurs avec les spectres de fréquences locales caractéristiques du modèle de référence donné et transmettant les résultats, déterminés sur la base de cette comparaison, à un dispositif d'entrée/sortie (6).

4. Procédé selon la revendication 3, caractérisé en ce qu'on utilise comme source lumineuse (3, 19), une source de lumière froide.

5. Procédé selon la revendication 3 ou 4, caractérisé en ce qu'on utilise comme source lumineuse une source lumineuse annulaire (19).

6. Procédé selon la revendication 3, caractérisé en ce que la source lumineuse (3) et l'unité à capteur (4) sont placées de manière spectralement sélective par rapport aux matériaux (1) à analyser.
